# EUROPEAN PATENT SPECIFICATION

(11) **EP 1 663 257 B1**
(45) Date of publication and mention of the grant of the patent: **07.01.2009**
(21) Application number: 04756926.4
(22) Date of filing: 09.07.2004
(51) Int. Cl.: A61K 31/7016, A61K 31/717, A61K 31/77, A61K 31/78, A61K 36/00, A61K 33/06, A61P 1/10

(54) **USE OF LAXATIVES FOR TREATING IRRITABLE BOWEL SYNDROME**
VERWENDUNG VON ABFÜHRMITTEL ZUR BEHANDLUNG DES REIZDARMSYNDROMS
UTILISATION DES LAXATIFS DANS LE TRAITEMENT DU SYNDROME DU COLON IRRITABLE

(30) Priority: 09.07.2003 US 485797 P
(43) Date of publication of application: 07.06.2006
(73) Proprietor: BRAINTREE LABORATORIES, INC., Braintree, MA 02185 (US)
(72) Inventor: PELHAM, Russell, W., Duxbury, MA 02332 (US); CLEVELAND, Mark, VB, Duxbury, MA 02331 (US); DIPALMA, Jack, A., Mobile, AL 36608 (US)
(74) Representative: Grund, Martin
(86) International application number: PCT/US2004/022392
(87) International publication number: WO 2005/007170

(56) References cited:
- EP-A- 0 488 139
- WO-A-97/03685
- WO-A-97/06808
- WO-A-03/099311
- G. F. LONGSTRETH ET AL: "Functional Bowel Disorders" GASTROENTEROLOGY, vol. 130, no. 4, April 2006 (2006-04), pages 1480-1491,

## Description

### BACKGROUND OF THE INVENTION

### Field of the Invention

This invention relates generally to gastroenterology and medicine. More particularly, the invention is directed to the therapeutic treatment of irritable bowel syndrome, as well as palliative treatment to reduce the frequency or severity of the appearance of the symptoms of irritable bowel syndrome.

### Summary of the Related Art

Irritable Bowel Syndrome (IBS) is a common disorder of the intestines that leads to cramping pain, flatulence, bloating, and changes in bowel habits. Some people with IBS have constipation (i.e., difficult or infrequent bowel movements); others have diarrhea (i.e., frequent loose stools, often with an urgent need to move the bowels); and some people experience both, alternating between constipation and diarrhea. IBS affects between 25 and 55 million people in the United States, and results in 2.5 to 3.5 million yearly visits to physicians. The symptom prevalence is approximately equally divided among the three IBS groups. The type and severity of symptoms associated with IBS varies widely. More than 40% of IBS patients have symptoms so severe that they have to take time off from work, curtail their social life, avoid sexual intercourse, cancel appointments, stop traveling, take medication, and even stay confined to their house for fear of embarrassment. The estimated health care cost of IBS in the United States is $8 billion per year (Talley et al., Gastroenterol., 109, 1736, (1995)).

The diagnostic algorithm for evaluating a patient with IBS usually follows the "Rome II" criteria. (Rome II. The Functional Gastrointestinal Disorders. Diagnosis, Pathophysiology and Treatment: A Multinational Consensus, Drossman, et al., 2nd ed. USA: Degnon Associates, p. 360 (2000)). The ROME II diagnostic criteria for IBS are the presence for at least 12 weeks, which need not be consecutive, in the preceding 12 months of abdominal discomfort or pain that has at least two of following three features: 1) relieved by defecation; 2) onset associated with a change in frequency of stool; and/or 3) onset associated with a change in form (appearance) of stool. In addition to these criteria, ROME II recognizes supportive symptoms of IBS: 1) fewer than three bowel movements per week; 2) more than three bowel movements per day; 3) hard or lumpy stools; 4) loose (mushy) or watery stools; 5) straining during a bowel movement; 6) urgency (having to rush to have a bowel movement); 7) feeling of incomplete bowel movement; 8) passing mucus (white material) during a bowel movement; and/or 9) abdominal fullness, bloating or swelling.

IBS patients can be divided into those suffering from diarrhea-predominant IBS, those suffering from constipation-predominant IBS, and those who alternate between these two groups. Muscles in the bowel normally contract a few times each day, moving feces along the bowel, while fluids and nutrients are reabsorbed at an appropriate rate, ultimately resulting in a bowel movement. Normal motility is achieved by these regular involuntary contractions of the colon with six to eight contractions being considered normal. However, a person with constipation-predominant IBS may have only one or two contractions per day, resulting in hard, lumpy stools due to increased water reabsorption. Patients with diarrhea-predominant IBS have as many as 25 contractions per day resulting in loose, watery stools due to decreased water reabsorption.

Without being bound by any particular theory, it is believed that in a person with IBS, the muscles of the bowel are exceptionally sensitive to stimuli, or "triggers" which further effects the number of contractions per day (Camilleri, et al., Aliment Pharmacol. Ther., 16, 1407-1430, (2002)). Although food or stress would not normally affect those not suffering from IBS, these triggers may provoke a strong intestinal response in a person suffering from IBS. For example, a person who does not have IBS may eat a salad or drink coffee without difficulties, while a person with IBS exhibits symptoms such as pain, bloating, and diarrhea upon consuming such foods. Many people with IBS report that symptoms frequently occur shortly after, or even during, meals. Not every person with IBS responds symptomatically to the same stimuli. The range of triggers is unique to each individual, although there are many common elements among most people with IBS. Symptoms may also be intermittent, and the symptoms associated with a given food or emotional state may change over time.

Treatments for IBS to date include dietary and lifestyle changes, stress reduction, and medications. Treatment of IBS is frequently an *ad hoc* matter, as each patient independently copes with symptoms and learns how to avoid attacks. None of these treatment regimens has provided lasting benefits to patients. Medications prescribed for IBS include anticholinergics, antispasmodics, antidiarrheals, and antidepressants. Patients are often advised to reduce exposure to foods that have triggered symptoms in the past and to reduce stress. Patients have also been advised to increase their consumption of dietary fiber, although they may be simultaneously advised to avoid using laxatives, which frequently contain fiber. More specifically, physicians often recommend that a patient consume precisely enough fiber to maintain soft, easily passed, stools. This is, of course, a difficult balance to achieve while eating a varied diet. Additionally, high-fiber diets may cause gas and bloating, which are themselves symptoms of IBS and can exacerbate IBS symptoms. Managing IBS has, therefore, involved carefully titrating the use of fiber laxatives to balance the colon function in the range between constipation and diarrhea. This control is imperfect at best, and varying the dose and frequency of use of fiber laxatives typically results in a patient oscillating between constipation and diarrhea, while enduring gas, bloating or abdominal pain. In fact, current medical consensus is that fiber has no benefit (Evidence-Based Position Statement on the Management of Irritable Bowel Syndrome in North America, Am. J. Gastroenterol., 97, S1-S5, (2002); Brandt, et al. Systematic Review on the Management of Irritable Bowel Syndrome in North America, Am. J. Gastroenterol., 97, S6-S26, (2002)).

More recently has the American College of Gastroenterology concluded that two medications, alosetron and tegaserod, have sufficient evidence for their efficacy in treating diarrhea-predominant IBS or constipation-predominant IBS, respectively. However, the use of alosetron is severely limited by its toxicity and the efficacy of tegaserod is small, e.g., only about one of eight patients treated with tegaserod had significant improvement in IBS symptoms compared with patients using placebo (Evidence-Based Position Statement on the Management of Irritable Bowel Syndrome in North America, Am. J. Gastroenterol., 97, S1-S5, (2002)).

Thus, the American Academy of Family Physicians counsels against the first line use of medications to treat IBS, and instead advocates eating a healthy diet, avoiding foods that seem to make one feel worse, and finding ways to handle stress (Viera, et al., American Family Physician 1867 (2002)). Therefore, it appears that, since there is no single medical treatment that safely and adequately improves the symptoms of patients with either diarrhea-predominant IBS, constipation-predominant IBS, or patients who alternate between these two forms, a medical need exists for such an effective treatment.

[WO 97/03685 describes laxative/antidiarrheal compositions comprising an osmotic laxative (PEG) and a fibre.]

### SUMMARY OF THE INVENTION

Disclosed is the use of a mixture formulated with an osmotic laxative and fiber, for the maunfacturing of a medicament for treating irritable bowel syndrome in mammals, wherein the medicament is to be administered in a therapeutically effective amount and regimen to a patient in need of such treatment.

It has been discovered that a formulation comprising an osmotic laxative, such as, for example, polyethylene glycol (PEG), with a laxative fiber, provides relief from IBS by simultaneously ameliorating both constipation and diarrhea. While patients have, in the past, been cautioned to avoid prolonged use of laxatives, patients with constipation-predominant IBS often require the use of laxatives for periods of time longer than those recommended.

In one aspect, the invention provides the claimed use for treating IBS, comprising administering a therapeutically effective regimen of a formulation comprising an osmotic laxative and fiber to a patient in need of such treatment. In some embodiments, the osmotic laxative is selected from the group consisting of magnesium sulphate, magnesium hydroxide, magnesium citrate, sodium phosphate, sodium sulphate, potassium sodium tartrate, lactulose, sorbitol, mannitol, glycerin and polyethylene glycol. In other embodiments, the osmotic laxative is a polyethylene glycol. In some embodiments, the polyethylene glycol has an average molecular weight in the range between about 2,000 daltons ("D") and about 10,000 D. In another embodiment, the osmotic laxative is a polyethylene glycol having an average molecular weight in the range of between about 3,000 D and about 4,000 D. In particular embodiments, the osmotic laxative is a polyethylene glycol having an average molecular weight of about 3,350 D. In other particular embodiments, the osmotic laxative is a polyethylene glycol having an average molecular weight of about 4,000 D. In some embodiments, the concentration of the osmotic laxative ranges from about 0.01 % to about 99% by weight of the total formulation. In other embodiments, the formulation contains from about 1% to about 40% by weight of osmotic laxative. In particular embodiments, the amount of osmotic laxative by weight ranges from about 2% to about 20%, or from about 5% to about 15%.

In some embodiments, the fiber is selected from the group consisting of psyllium fiber, ispaghula, calcium polycarbophil, guar gum, cellulose, methylcellulose and combinations thereof. In particular embodiments, the fiber is selected from the group psyllium fiber and cellulose. In one embodiment, the fiber is psyllium. In another embodiment, the fiber is cellulose. In some embodiments, the concentration of the fiber ranges from about 0.01% to about 99% by weight of the total formulation. In other embodiments, the formulation contains from about 1% to about 40% by weight of fiber. In particular embodiments, the amount of fiber by weight ranges from about 2% to about 20%, or from about 5% to about 15%.

In some embodiments, the use comprises administration of a formulation in which the osmotic laxative and fiber are present in a ratio between about 3:1 and about 1:3 by weight. In certain embodiments, the method comprises administration of a formulation in which the osmotic laxative and fiber are present in a ratio of about 1:1 by weight. In one particular embodiment, the method comprises administration of a formulation comprising about 17 grams of PEG 3350 and about 3 grams to about 6 grams of psyllium. In another particular embodiment, the method comprises administration of a formulation comprising about 17 grams of PEG 3350 and about 15 grams to about 24 grams of cellulose. In some embodiments, the osmotic laxative and the fiber are administered separately as different formulations.

In some embodiments, the formulation or formulations are administered orally. In other embodiments the formulation or formulations is/are administered through a nasogastric tube. In still other embodiments, the formulation or formulations is/are administered once daily. In some embodiments, the formulation or formulations is/are administered at least twice daily. In some embodiments, the formulation or formulations is/are administered up to about 12 weeks.

### DETAILED DESCRIPTION

It has been discovered that administering an osmotic laxative and fiber to a patient effectively treats IBS and alleviates the symptoms associated with IBS. A therapeutically effective regime includes administering the osmotic laxative and the fiber separately or together in an amount and at a frequency and for a period of time sufficient to treat irritable bowel syndrome such that the symptoms of IBS are reduced or eliminated.

Thus, the present invention includes, at least in part, the use of an osmotic laxative and fiber, for the preparation of a medicament for treating IBS, wherein the medicament istobe administered in a therapeutically effective amount to a patient in need thereof. As used herein, the term "irritable bowel syndrome" or "IBS" is meant to encompass diarrhea-predominant IBS, constipation-predominant IBS, and the condition that alternates between these two forms, unless specified as one of these conditions.

As used herein the term "therapeutically effective" when used in connection with the method provided by the invention, means sufficient to reduce or eliminate the symptoms of irritable bowel syndrome or to provide symptomatic or palliative relief to the patient.

As used herein the term "to treat" when used in connection with the use provided by the invention, means to reduce or eliminate the symptoms of irritable bowel syndrome or to provide symptomatic or palliative relief to the patient, through the use of a formulation comprising an osmotic laxative and fiber.

Useful osmotic laxatives include, but are not limited to, poorly absorbed ions such as magnesium sulphate (e.g., Epsom salt, Humco Epsom Salt, Humco Corp, Texarkana, TX), magnesium hydroxide (e.g., Phillips Milk of Magnesia^{®}, Bayer Corporation, Morristown, NJ ), magnesium citrate (e.g., magnesium citrate, Oral Solution, Lemon Flavor Laxative, Valu-Rite Products, McKesson Corp., San Francisco, CA), sodium phosphate (e.g., Fleet's PhosPho-Soda, Lynchburg, Virginia), sodium sulphate (e.g., Glauber's salt, US Trading & Marketing LLC, Washington, D.C.,), potassium sodium tartrate (e.g., Rochelle salt, Westco, Inc., North Hollywood, CA), and poorly absorbed disaccharides, including, but not limited to, lactulose (e.g., Dulcolax^{®}, Boehringer Ingelheim Consumer Healthcare Ridgefield, CT), sorbitol, mannitol and glycerin.

Another useful osmotic laxative is polyethylene glycol ("PEG") (available, for example, from Dow Chemical Company, Midland, MI). When administered to mammals, PEG softens the stool, increases the frequency of bowel movements by attracting and retaining water in the stool, and improves bowel motility and stool formation. When used as a laxative, PEG is commonly administered orally after being dissolved in, e.g., about 8 ounces of water, juice, or other liquid.

PEG is a solid or liquid at room temperature depending upon its molecular weight. PEGs are identified by a number that identifies the average molecular weight of the polymer. PEGs having an average molecular weight in the range of about 1,000 D to about 25,000 D (i.e., PEG 1000 - PEG 25000) may be used as osmotic agents to soften mammalian stools.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%.

In the interest of convenience, the formulations disclosed herein include PEGs that are solid at room temperature. According to some embodiments, the PEGs included in the formulations have an average molecular weight in the range of about 2,000 D to about 10,000 D (*i*.*e*., PEG 2000 - PEG 10000), or between about 3,000 D and about 4,000 D (*i*.*e*., PEG 3000 - PEG 4000). In particular embodiments, PEG 3350, a polyglycol having an average molecular weight of 3,350, is used. In other particular embodiments, PEG 4000 is used. As a matter of manufacturing convenience, in at least some embodiments, the actual molecular weight of the PEG in the mixture is not less than 90% and not greater than 110% of the nominal value.

The use of the present invention further includes administering fiber, As used herein the term "fiber" or "dietary fiber" is defined as the polysaccharides and lignins that are resistant to hydrolysis by the digestive enzymes in humans. Fiber includes products containing both soluble and insoluble fiber. Commercial preparations of psyllium husk, such as, for example, Metamucil^{®} (Proctor and Gamble, Cincinnati, OH), may also be used in preparing the therapeutic formulations disclosed herein. Dietary fiber has a laxative effect with whole-grain breads and cereals, beans, fruits, and vegetables being good dietary sources of fiber. High-fiber diets keep the colon mildly distended, which is believed to help to prevent spasms from developing in the bowel. Some forms of fiber also keep water in stools, thereby preventing the formation of hard stools that are difficult to pass.

There are two general types of fiber: soluble and insoluble. Soluble fibers are designated as such because they are viscous, gel-forming fibers. Soluble fiber absorbs water and makes stools softer and easier to pass. Soluble fiber is found in oats, legumes, certain fruits, and psyllium. Insoluble fibers do not form gels. Insoluble fiber is used to treat constipation, but often makes diarrhea worse. Insoluble fiber is found in fruits, vegetables, whole grain breads, and cereals.

Most natural fibers are actually a mixture of both soluble and insoluble fiber. For example, psyllium fiber is rich in soluble fiber (approximately 70%), wheat bran contains mostly insoluble fiber, while oat bran contains about 7% soluble fiber.

Three commercially available kinds of fiber are currently approved for medical use and are suitable for inclusion in the therapeutic formulations of the present invention: psyllium, (e.g., Metamucil^{®}, Proctor and Gamble, Cincinnati, OH; Konsyl^{®}, Konsyl Pharmaceuticals, Edison, NJ) partially-hydrolyzed guar gum (e.g., Benefiber^{®}, Novartis Consumer Health, Parsippany, NJ), and cellulose (e.g., Unifiber^{®}, Niche Pharmaceuticals, Roanoke TX). Amounts of fibers that can be used vary according to the type of fiber. For example, amounts of the various fibers used in the method provided by the present invention include, but are not limited to, about 1 gram to about 12 grams of psyllium, and about 3 grams to about 9 grams of psyllium; and about 6 grams of psyllium; about 2.5 grams to about 30 grams of partially hydrolyzed guar gum; and about 2 grams to about 30 grams of cellulose; and about 10 to about 20 grams of cellulose; or about 15 grams of cellulose.

The osmotic laxative and the fiber are present in the formulation in a laxative-to-fiber ratio between about 3:1 and about 1:3 by weight. For example, the osmotic laxative and the fiber may be present in the composition in approximately equal amounts, *i*.*e*., in a laxative-to-fiber ratio of about 1:1 by weight.

To prepare a therapeutic formulation in solution form, at least one of the aforementioned osmotic laxatives is dissolved in a pharmaceutically acceptable vehicle, such as, for example, water, vegetable or fruit juices, soda, coffee, tea, dairy, soy, or other beverage, an electrolyte solution containing for example, single electrolytes or mixtures thereof, including physiological electrolytes such as sodium, potassium, chloride, bicarbonate, or phosphate or combinations of these beverages and electrolytes.

For example, if PEG is used, the concentration of PEG in the therapeutic formulation ranges from about 0.01% to about 99% by weight of the total composition. For example, the composition can contain from about 1% to about 40% by weight of PEG. In particular embodiments, the amount of PEG by weight ranges from about 2% to about 20%, or from about 5% to about 15%. The specific concentration of PEG used in each formulation may be increased or decreased as necessary to provide relief while avoiding discomfort or urgency.

The fiber may then admixed into the solution. The concentration of the fiber ranges from about 0.01% to about 99% by weight of the total composition. For example, the composition can contain from about 1% to about 40% by weight of fiber. In particular embodiments, the amount of fiber by weight ranges from about 2% to about 20%, or from about 5% to about 15%. The specific concentration of fiber used in each formulation may be increased or decreased as necessary to provide relief while avoiding discomfort or urgency. For example, this invention may utilize a formulation described in U.S. Patent No. 5,710,183 to treat irritable bowel syndrome.

Alternatively, the osmotic laxative and fiber can be administered in separate solutions within about 30 minutes of each other, in any order. When taking the laxative and fiber separately, the laxative-to-fiber ratio is usually between about 3:1 and about 1:3 by weight. In particular examples, the osmotic laxative and the fiber are taken in approximately equal amounts, *i*.*e*., in alaxative-to-fiber ratio of about 1:1 by weight. The concentration of the osmotic laxative solution and the concentration of the fiber solution range from about 0.01% to about 99% by weight. For example, the solution of either the osmotic laxative or fiber contains from about 1% to about 40% by weight of fiber. In particular examples, the amount of fiber or osmotic laxative by weight ranges from about 2% to about 20%, or from about 5% to about 15%.

The formulations useful in the use of the present invention can also contain any number of different additives. For example, a wetting agent and/or other ingredients, such as, for example, simethicone, may be added to the formulation to make the mixture flow freely or for other purposes. Additionally or alternatively, the formulation can contain flavorings such as cherry, grape, tea, apple, lemon-lime flavoring, etc., which may be oil-based. Such flavorings are commercially available from, *e*.*g*., IFF (International Flavors and Fragrances, Chicago, IL), or Flavors of North America, (Carol Stream, IL), or Kraft Foods, (Glenview, IL) or other vendor of food/pharmaceutical grade flavors. The formulation can also or alternatively contain sweeteners such as sugar, sucralose, acesulfameK, fructose, and/or aspartame, which are also commercially available, *e*.*g*., from Spectrum Quality Products, New Brunswick, New Jersey, or McNeil Nutritionals Division of McNeil-PPC. Inc., Fort Washington, PA. or other vendor of food/pharmaceutical grade chemicals. Flavor enhancers such as, but not limited to, malic acid citric acid, and/or ascorbic acid can be added. These enhancers are available from, *e*.*g*., Spectrum Quality Products, New Brunswick, NJ, or other vendor of food/pharmaceutical grade chemicals. The formulation can also be colored to match the flavor, *e*.*g*., light brown for apple juice, dark brown for tea, purple for grape, etc. Useful colorings can be commercially obtained, *e*.*g*., from Warner-Jenkinson, St. Louis, MO, or other vendors of food/pharmaceutical grade colors. Preservatives can be added to keep freshness. Some useful preservatives include, but are not limited to, parabens, benzoates, sorbates, and alcohols, obtainable from, *e*.*g*., Spectrum Quality Products, New Brunswick, NJ, or other vendors of food/pharmaceutical grade chemicals. The formulation may be clear or unclear (cloudy, a suspension, etc.) with additives for product effect to look like orange juice, iced tea, and other drinks. Other additives can be used to optimize taste, odor, stability, solubility, acidity, color, etc. of the formuation. See, *e*.*g*., (International Flavor and Fragrances, Chicago, IL, or Spectrum Quality Products, New Brunswick, NJ, or other vendor of food/pharmaceutical grade chemicals).

Other medications may also be included in the formulation(s) such as those known in the art, such as antimicrobials such as cephalosporins (*e*.*g*. Keflex^{®}, cephalexin, Dista Products Company, Indianapolis, IN), penicillins (*e*.*g*. Pfizerpen^{®}, penicillin G potassium, Pfizer Inc, NY, NY) erythromycins (*e*.*g*. ERY-TAB^{®}, erythromycin delayed-release tablets, Abbott Laboratories, Chicago, IL), tetracyclines (*e*.*g*. doxycycline monohydrate capsules, Watson Lab., Inc., Corona, CA; UROBIOTIC^{®}-250, oxytetracycline hydrochloride, Pfizer, Pfizer Inc, NY, NY), antifungal agents (*e*.*g*. GRIFULVIN^{®}, griseofulvin tablets, Ortho Dermatological, Skillman, NJ; NIZORAL^{®}, ketoconazole, Janssen Titusville, NJ), antiviral agents (*e*.*g*. SYMMETREL^{®}, amantadine hydrochloride, Endo Labs, Chadds Ford, PA); EPIVIR-HBV^{®}, lamivudine, GlaxoSmithKline, Philadelphia, PA), anti-inflammatories (*e*.*g*. Advil^{®}, ibuprofen, Wyeth Labs, Madison, NJ; Celebrex^{®}, celecoxib, G.D. Searle LLC Chicago IL), anti-cancer (*e*.*g*. ADRIAMYCIN RDF^{®}, doxorubicin hydrochloride, Pfizer, Inc. New York, NY; ELOXATIN™, oxaliplatin, Sanofi-Synthelabo Inc. NY, NY), anti-hypertensives (*e*.*g*. INDERAL^{®}, propranolol hydrochloride LA, Wyeth-Ayerst, Philadelphia, PA; DIBENZYLINE^{®}, phenoxybenzamine hydrochloride, WellSpring, Neptune, NJ; DIOVAN HCT^{®}, valsartan and hydrochlorothiazide, Novartis, Parsippany, NJ), antipsychotics (*e*.*g*. HALDOL^{®}, haloperidol, Ortho-McNeil, Raritan, NJ; CLOZARIL^{®}, clozapine, Novartis, Parsippany, NJ), gastrointestinal agents (*e*.*g*. LOTRONEX^{®},' alosetron hydrochloride, GlaxoSmithKline, Philadelphia, PA; Gas-X Extra Strength Chewable Tablets^{®}, simethicone, Novartis Consumer Products, Parsippany, NJ; ANZEMET^{®}, dolasetron mesylate, Aventis, Bridgewater, NJ; ASACOL^{®}, mesalamine, Procter & Gamble Pharmaceuticals, Mason, OH) or other agents, the list given here not being meant to be all-inclusive of the possible drug classes or specific agents.

For ease of administration, the therapeutic formulation(s) may also be formulated as gels. A typical gel composition includes at least one aforementioned osmotic laxative, such as PEG and one fiber dissolved in an aqueous mixture. A gelling agent, such as hydroxyethylcellulose, hydroxypropylcellulose, or hydroxypropylmethylcellulose (AQUALON CO., Hopewell, Virginia) is then added to the mixture with agitation. According to at least some embodiments, the concentration of the gelling agent ranges from 0.1% to about 4.0% by weight of the total composition.

Alternatively, the therapeutic formulation(s) may be formulated as a dry powder. The dry powder is dissolved in water or another liquid before it is administered to a patient. In the case of dry powder mixing, the formulation contains between about 1% to about 99% osmotic laxative and between about 1% to about 99% fiber. The weight ratio of osmotic laxative to fiber is preferably in the range of about 3:1 to about 1:2. The dry powder may be compressed into tablets or food products such as confections or bakery goods for oral administration to patients.

The osmotic agent and the fiber may be pre-mixed in any therapeutically desirable ratio and sold to patients, or the formulation may be prepared by a patient or caregiver immediately prior to its administration. The solution may be administered orally or through a nasogastric tube.

The term of treatment necessary to provide lasting relief from the symptoms of IBS varies among individuals. For example, one dose of the osmotic laxative/fiber formulation is taken each day. If necessary, two or more doses may be taken to obtain relief. Some patients may obtain relief in as few as one to three days, while for others a longer course of treatment may be required, e.g., up to about 12 weeks. The course of treatment is continued until the spasms of the colon that are characteristic of IBS cease.

After using the formulations of the present invention in a course of treatment of up to about 12 weeks, patients demonstrated substantial relief from the pain, constipation, and diarrhea that are common symptoms of IBS. By increasing the amount of water retained in the stool, retaining stool form, and soothing the bowel by keeping it moderately distended for a sufficient period of time, the formulation and method disclosed herein not only control IBS but, frequently, eliminate recurrent spasms. Although not wishing to be bound by any particular theory, it is thought that the osmotic effect of the PEG laxative and the fiber keep stools soft and moving, and also forms a gel which acts to contain excess water. This may slightly distend the bowel, reducing spasms.

The following nonlimiting examples further illustrate certain embodiments of the present invention:

### EXAMPLE 1: THERAPEUTIC FORMULATIONS

### Formulation 1

17 grams of PEG 3350 (trade name MiraLax,^{®}, Braintree Laboratories, Braintree, MA) and approximately 24 grams of Metamucil® fiber are mixed in 300 ml of water and stirred.

### Formulation 2

20 grams of Macrogol^{®} 4000, a PEG having an average molecular weight of about 4000 (BASF, Toronto, Canada), is mixed with approximately 24 grams of Metamucil^{®} fiber (Proctor & Gamble, Cincinnati, OH) in about 300 ml of an electrolyte solution including approximately 125 mEq/l Na⁺, 10 mEq/l K⁺, 35 mEq/l Cl⁻, 20 mEq/l HCO₃⁻, and 80 mEq/l SO₄²⁻ and stirred at room temperature. Exemplary electrolytes can be obtained from Morton Salt, Mallinckrodt of St. Louis MO, Spectrum Quality Products of New Brunswick NJ, or other vendors of food/pharmaceutical grade chemicals.

### Formulation 3

17 grams of PEG 3350 (trade name MiraLax^{®}, Braintree Laboratories, Braintree, MA) and approximately 20 grams of partially hydrolyzed guar gum fiber (Benefiber^{®}, Novartis Consumer Health, Parsippany, NJ) are mixed in 300 ml of water and stirred at room temperature.

### Formulation 4

20 grams of Macrogol^{®} 4000 (BASF, Toronto, Canada) is mixed with approximately 20 grams of partially hydrolyzed guar gum fiber (Benefiber^{®}, Novartis Consumer Health, Parsippany, NJ) fiber in about 300 ml of an electrolyte solution including approximately 125 mEq/l Na⁺, 10 mEq/l K⁺, 35 mEq/l Cl⁻, 20 mEq/l HCO₃⁻, and 80 mEq/l SO₄²⁻ and stirred at room temperature.

### Formulation 5

About 17 grams of PEG 3350 (trade name MiraLax^{®}, Braintree Laboratories, Braintree, MA) and approximately 20 grams of cellulose (trade name UniFiber^{®}, Niche Pharmaceuticals, Roanoke TX) are mixed in 300 ml of juice and stirred at room temperature.

### Formulation 6

20 grams of sodium phosphate (Fleet's PhosPho-Soda, Lynchburg, Virginia) is mixed with approximately 24 grams of Metamucil^{®} fiber in about 300 ml of a solution at room temperature.

### Formulation 7

5 grams of lactulose (Dulcolax^{®}, Boehringer Ingelheim Consumer Healthcare, Ridgefield, CT) and approximately 20 grams of cellulose are mixed in 300 ml of milk and ice water and mixed in a blender.

### EXAMPLE 2: CASE STUDIES

### Case Study 1

A 27-year-old female reported 18 months of chronic abdominal pain. Clinical evaluations showed that she met the Rome II criteria for IBS and had constipation-predominate stool habits. A hysterectomy two months prior exacerbated her symptoms. The patient was instructed to take about 17 grams of PEG 3350 (trade name MiraLax,^{®}, Braintree Laboratories, Braintree, MA) and approximately 24 grams of Metamucil® fiber, once daily. The patient reported significant clinical improvement with increased number of stool after 4 days, less straining during a bowel movement, fewer hard or lumpy stools, and abdominal fullness, bloating and swelling were ameliorated. The patient's symptoms did not recur.

### Case Study 2

A 60-year-old female with life-long constipation and abdominal pain met the Rome II criteria for constipation-predominant IBS. The patient had tried several treatment regimens without success, including bulk laxatives, laxatives, mineral oil, and cisapride. Treatment with one dose of MiraLax^{®} alone (51 grams in 500 ml) resulted in some improvement with more bowel movements. The patient was placed on a once daily dose of about 17 grams of PEG 3350 (trade name MiraLax,^{®}, Braintree Laboratories, Braintree, MA) and approximately 24 grams of Metamucil® fiber. The patient reported significant clinical improvement with more stools within 3-4 days, less straining during a bowel movement, fewer hard or lumpy stools, and abdominal fullness, bloating and swelling were ameliorated. The patient reported that she was without symptoms after taking the formulation for 8 weeks.

### Case Study 3

A 30-year-old female reported 18 months of chronic abdominal pain. Clinical evaluations showed that she met the Rome II criteria for IBS and had diarrhea-predominant stool habits. The patient started on about 17 grams of PEG 3350 (trade name MiraLax,^{®}, Braintree Laboratories, Braintree, MA) and approximately 24 grams of Metamucil® fiber, once daily and reported significant clinical improvement with symptoms such as more than three bowel movements per day, loose (mushy) or watery stools, urgency, and abdominal fullness, bloating and swelling were ameliorated within 7 days. The patient's symptoms did not recur.

### Case Study 4

A patient presenting with constipation-predominant IBS is given any formulation selected from Formulations 1-7 once daily for up to 12 weeks. Symptoms such as fewer than three bowel movements per week, straining during a bowel movement, hard or lumpy stools, and abdominal fullness, bloating or swelling are relieved or ameliorated.

### Case Study 5

A patient presenting with diarrhea-predominant IBS is given any formulation selected from Formulations 1-7 once daily for up to 12 weeks. Symptoms such as more than three bowel movements per day, loose (mushy) or watery stools, urgency (having to rush to have a bowel movement), and abdominal fullness, bloating or swelling are relieved or ameliorated.

### Case Study 6

A patient presenting with alternating constipation-predominant IBS symptoms and diarrhea-predominant IBS symptoms is given any formulation selected from Formulations 1-7 once daily for up to 12 weeks. Symptoms such as more than three bowel movements per day, loose (mushy) or watery stools, urgency (having to rush to have a bowel movement), fewer than three bowel movements per week, straining during a bowel movement, hard or lumpy stools and abdominal fullness, bloating or swelling are relieved or ameliorated.

### Case Study 7

A patient presenting with constipation-predominant IBS is given any formulation selected from Formulations 1-7 at least twice daily for up to 12 weeks. Symptoms such as fewer than three bowel movements per week, hard or lumpy stools, straining during a bowel movement and abdominal fullness, bloating or swelling are relieved or ameliorated.

### Case Study 8

A patient presenting with diarrhea-predominant IBS is given any formulation selected from Formulations 1-7 at least twice daily for up to 12 weeks. Symptoms such as more than three bowel movements per day, loose (mushy) or watery stools, urgency (having to rush to have a bowel movement), and abdominal fullness, bloating or swelling are relieved or ameliorated.

### Case Study 9

A patient presenting with alternating constipation-predominant IBS symptoms and diarrhea-predominant IBS symptoms is given any formulation selected from Formulations 1-7 at least twice daily for up to 12 weeks. Symptoms such as more than three bowel movements per day, loose (mushy) or watery stools, urgency (having to rush to have a bowel movement), fewer than three bowel movements per week, straining during a bowel movement, hard or lumpy stools and abdominal fullness, bloating or swelling are relieved or ameliorated.

## Claims

1. Use of an osmotic laxative and a fiber for the preparation of a therapeutic formulation for treating irritable bowel syndrome

2. The use of claim 1, wherein the osmotic laxative is selected from the group consisting of magnesium sulphate, magnesium hydroxide, magnesium citrate, sodium phosphate, sodium sulphate, potassium sodium tartrate, lactulose, sorbitol, mannitol, glycerin and polyethylene glycol.

3. The use of claim 1, wherein the osmotic laxative is a polyethylene glycol.

4. The use of claim 3, wherein the osmotic laxative is a polyethylene glycol having an average molecular weight of 2,000 D to 10,000 D.

5. The use of claim 4, wherein the osmotic laxative is a polyethylene glycol having an average molecular weight of 3,000 D to 4,000 D.

6. The use of claim 3, wherein the osmotic laxative is polyethylene glycol 3350.

7. The use of claim 3, wherein the osmotic laxative is polyethylene glycol 4000.

8. The use of claim 1, wherein the fiber is selected from the group consisting of psyllium fiber, ispaghula, calcium polycarbophil, guar gum, cellulose, methylcellulose, and combinations thereof.

9. The use of claim 1, wherein the fiber is selected from the group consisting of psyllium fiber and cellulose.

10. The use of claim 1, wherein the fiber is psyllium fiber.

11. The use of claim 1, wherein the fiber is cellulose.

12. The use of claim 1, wherein the formulation is to be administered orally.

13. The use of claim 12, wherein the formulation is to be administered once daily.

14. The use of claim 12, wherein the formulation is to be administered at least twice daily.

15. The use of claim 12, wherein the osmotic laxative and fiber-bulking are present in the formulation in a ratio of 3:1 by weight.

16. The use of claim 12, wherein the osmotic laxative and fiber are present in the formulation in a ratio of 1:3 by weight.

17. The use of claim 12, wherein the osmotic laxative and fiber are present in the formulation in a ratio of 1:1 by weight.

18. The use of claim 1, wherein the osmotic laxative is present in the formulation in a concentration of 1% to 40% by weight.

19. The use of claim 18, wherein the osmotic laxative is present in the formulation in a concentration of 2% to 20% by weight.

20. The use of claim 18, wherein the osmotic laxative is present in the formulation in a concentration of 5% to 15% by weight.

21. The use of claim 1, wherein the fiber agent is present in the formulation in a concentration of 1% to 40% by weight.

22. The use of claim 21, wherein the fiber is present in the formulation in a concentration of 2% to 20% by weight.

23. The use of claim 21, wherein the fiber is present in the formulation in a concentration of 5% to 15% by weight.

24. The use of claim 1, wherein the formulation comprises 17 grams of PEG 3350 and 3 grams to 6 grams of psyllium.

25. The use of claim 24, wherein the formulation is to be administered once daily for up to about 12 weeks.

26. The use of claim 1, wherein the formulation comprises 17 grams of PEG 3350 and 15 grams to 24 grams of cellulose.

27. The use of claim 26, wherein the formulation is to de administered once daily for up to about 12 weeks.

## Patentansprüche

1. Verwendung eines osmotischen Laxativs und einer Faser zur Herstellung einer therapeutischen Formulierung zur Behandlung des Reizdarmsyndroms.

2. Die Verwendung nach Anspruch 1, wobei das osmotische Laxativ ausgewählt ist aus der Gruppe bestehend aus Magnesiumsulfat, Magnesiumhydroxid, Magnesiumcitrat, Natriumphosphat, Natriumsulfat, Kaliumnatriumtartrat, Lactulose, Sorbitol, Mannitol, Glyzerin und Polyethylenglykol.

3. Die Verwendung nach Anspruch 1, wobei das osmotische Laxativ ein Polyethylenglykol ist.

4. Die Verwendung nach Anspruch 3, wobei das osmotische Laxativ ein Polyethylenglykol mit einem durchschnittlichen Molekulargewicht von 2.000 D bis 10.000 D ist.

5. Die Verwendung nach Anspruch 4, wobei das osmotische Laxativ ein Polyethylenglykol mit einem durchschnittlichen Molekulargewicht von 3.000 D bis 4.000 D ist.

6. Die Verwendung nach Anspruch 3, wobei das osmotische Laxativ Polyethylenglykol 3350 ist.

7. Die Verwendung nach Anspruch 3, wobei das osmotische Laxativ Polyethylenglykol 4000 ist.

8. Die Verwendung nach Anspruch 1, wobei die Faser ausgewählt ist aus der Gruppe bestehend aus einer Psylliumfaser, Ispaghula, Kalziumpolycarbophil, Guaran, Cellulose, Methylcellulose und Kombinationen davon.

9. Die Verwendung nach Anspruch 1, wobei die Faser ausgewählt ist aus der Gruppe bestehend aus einer Psylliumfaser und Cellulose.

10. Die Verwendung nach Anspruch 1, wobei die Faser eine Psylliumfaser ist.

11. Die Verwendung nach Anspruch 1, wobei die Faser Cellulose ist.

12. Die Verwendung nach Anspruch 1, wobei die Formulierung oral zu verabreichen ist.

13. Die Verwendung nach Anspruch 12, wobei die Formulierung einmal täglich zu verabreichen ist.

14. Die Verwendung nach Anspruch 12, wobei die Formulierung zumindest zweimal täglich zu verabreichen ist.

15. Die Verwendung nach Anspruch 12, wobei das osmotische Laxativ und der Faserfüllstoff in der Formulierung in einem Gewichtsverhältnis von 3:1 enthalten sind.

16. Die Verwendung nach Anspruch 12, wobei das osmotische Laxativ und die Faser in der Formulierung in einem Gewichtsverhältnis von 1:3 enthalten sind.

17. Die Verwendung nach Anspruch 12, wobei das osmotische Laxativ und die Faser in der Formulierung in einem Gewichtsverhältnis von 1:1 enthalten sind.

18. Die Verwendung nach Anspruch 1, wobei das osmotische Laxativ in der Formulierung in einer Konzentration von 1 bis 40 Gew. % enthalten ist.

19. Die Verwendung nach Anspruch 18, wobei das osmotische Laxativ in der Formulierung in einer Konzentration von 2 bis 20 Gew. % enthalten ist.

20. Die Verwendung nach Anspruch 18, wobei das osmotische Laxativ in der Formulierung in einer Konzentration von 5 bis 15 Gew. % enthalten ist.

21. Die Verwendung nach Anspruch 1, wobei das Faseragenz in der Formulierung in einer Konzentration von 1 bis 40 Gew. % enthalten ist.

22. Die Verwendung nach Anspruch 21, wobei die Faser in der Formulierung in einer Konzentration von 2 bis 20 Gew. % enthalten ist.

23. Die Verwendung nach Anspruch 21, wobei die Faser in der Formulierung in einer Konzentration von 5 bis 15 Gew. % enthalten ist.

24. Die Verwendung nach Anspruch 1, wobei die Formulierung 17 Gram PEG 3350 und 3 Gramm bis 6 Gramm Psyllium umfasst.

25. Die Verwendung nach Anspruch 24, wobei die Formulierung einmal täglich für bis zu ungefähr 12 Wochen zu verabreichen ist.

26. Die Verwendung nach Anspruch 1, wobei die Formulierung 17 Gramm PEG 3350 und 15 Gramm bis 24 Gramm Cellulose umfasst.

27. Die Verwendung nach Anspruch 26, wobei die Formulierung einmal täglich für bis zu ungefähr 12 Wochen zu verabreichen ist.

## Revendications

1. Utilisation d'un laxatif osmotique et d'une fibre pour la préparation d'une formulation thérapeutique pour le traitement d'un syndrome du côlon irritable.

2. L'utilisation selon la revendication 1, où le laxatif osmotique est choisi à partir du groupe composé de sulfate de magnésium , hydroxyde de magnésium , citrate de magnésium , phosphate de sodium , sulfate de sodium , tartrate de sodium et de potassium , lactulose , sorbitol, mannitol , glycérine et glycol de polyéthylène .

3. L'utilisation selon la revendication 1 , où le laxatif osmotique est un glycol de polyéthylène .

4. L'utilisation selon la revendication 3 , où le laxatif osmotique est un glycol de polyéthylène ayant un poids moléculaire moyen de 2000 D à 10000 D .

5. L'utilisation selon la revendication 4 , où le laxatif osmotique est un glycol de polyéthylène ayant un poids moléculaire moyen de 3000 D à 4000 D .

6. L'utilisation selon la revendication 3 , où le laxatif osmotique est du glycol de polyéthylène 3350 .

7. L'utilisation selon la revendication 3 , où le laxatif osmotique est du glycol de polyéthylène 4000 .

8. L'utilisation selon la revendication 1 , où la fibre est choisie à partir du groupe composé de la fibre de psyllium , ispaghuia , polycarbophile de calcium , gomme de guar , cellulose , méthylcellulose , et des combinaisons de ces composés .

9. L'utilisation selon la revendication 1 , où la fibre est choisie à partir du groupe composé de la fibre de psyllium et de la cellulose .

10. L'utilisation selon la revendication 1 , où la fibre est la fibre de psyllium .

11. L'utilisation selon la revendication 1 , où la fibre est la cellulose .

12. L'utilisation selon la revendication 1 , où la formulation doit être administrée de façon orale .

13. L'utilisation selon la revendication 12 , où la formulation doit être administrée une fois par jour .

14. L'utilisation selon la revendication 12 , où la formulation doit être administrée au moins deux fois par jour .

15. L'utilisation selon la revendication 12 , où le laxatif osmotique et le regroupement de fibres sont présents dans la formulation dans un ration 3 : 1 en poids .

16. L'utilisation selon la revendication 12 , où le laxatif osmotique et la fibre sont présents dans la formulation dans un ration 1 : 3 en poids .

17. L'utilisation selon la revendication 12 , où le laxatif osmotique et la fibre sont présents dans la formulation dans un ration 1 : 1 en poids .

18. L'utilisation selon la revendication 1 , où le laxatif osmotique est présent dans la formulation dans une concentration de 1 % à 40 % en poids .

19. L'utilisation selon la revendication 18 , où le laxatif osmotique est présent dans la formulation dans une concentration de 2 % à 20 % en poids .

20. L'utilisation selon la revendication 18 , où le laxatif osmotique est présent dans la formulation dans une concentration de 5 % à 15 % en poids .

21. L'utilisation selon la revendication 1 , où l'agent fibreux est présent dans la formulation dans une concentration de 1 % à 40 % en poids .

22. L'utilisation selon la revendication 21 , où la fibre est présente dans la formulation dans une concentration de 2 % à 20 % en poids .

23. L'utilisation selon la revendication 21 , où la fibre est présente dans la formulation dans une concentration de 5 % à 15 % en poids .

24. L'utilisation selon la revendication 1 , où la formulation comprend 17 grammes de PEG 3350 et 3 grammes à 6 grammes de psyllium .

25. L'utilisation selon la revendication 24 , où la formulation doit être administrée une fois par jour pendant jusqu'à environ 12 semaines .

26. L'utilisation selon la revendication 1 , où la formulation comprend 17 grammes de PEG 3350 et 15 grammes à 24 grammes de cellulose .

27. L'utilisation selon la revendication 26 , où la formulation doit être administrée une fois par jour pendant jusqu'à environ 12 semaines.
